**Europäisches Patentamt**

**European Patent Office**

**Office européen des brevets**

(19)

(11) Veröffentlichungsnummer: **0 229 639 B1**

(12) # EUROPÄISCHE PATENTSCHRIFT

(45) Veröffentlichungstag der Patentschrift: **07.04.93**

(51) Int. Cl.⁵: **A61F 13/46**, A61F 13/58

(21) Anmeldenummer: **87100155.8**

(22) Anmeldetag: **08.01.87**

(54) **Monatsbinde.**

(30) Priorität: **10.01.86 JP 1667/86 U**

(43) Veröffentlichungstag der Anmeldung:
**22.07.87 Patentblatt 87/30**

(45) Bekanntmachung des Hinweises auf die
Patenterteilung:
**07.04.93 Patentblatt 93/14**

(84) Benannte Vertragsstaaten:
**AT BE CH DE FR GB IT LI LU NL SE**

(56) Entgegenhaltungen:
**DE-A- 2 644 032**
**US-A- 4 376 440**

(73) Patentinhaber: **UNI-CHARM CORPORATION**
**182, Shimobun Kinsei-cho**
**Kawanoe-shi Ehime-ken(JP)**

(72) Erfinder: **Suzuki, Migaku**
**221-11, Shimobun Kinsei-cho**
**Kawanoe-shi Ehime-ken(JP)**
Erfinder: **Yamamoto, Masamitsu**
**2529-503, Aza-Miyashita Kawanoe-cho**
**Kawanoe-shi Ehime-ku(JP)**
Erfinder: **Ujimoto, Hiroshi**
**2350-1, Kawanoe-cho**
**Kawanoe-shi Ehime-ken(JP)**
Erfinder: **Tanaka, Yoshikazu**
**385-1-3, Handa-otsu Kaneda-cho**
**Kawanoe-shi Ehime-ken(JP)**
Erfinder: **Aono, Hiromi**
**3131-6, Kawanoe-cho**
**Kawanoe-shi Ehime-ken(JP)**

(74) Vertreter: **Sperling, Rüdiger, Dipl.-Ing. et al**
**Patentanwälte Dipl.Ing.S. Staeger**
**Dipl.Ing.Dipl.Wirtsch.Ing. R Sperling Müller-**
**strasse 31**
**W-8000 München 5 (DE)**

EP 0 229 639 B1

**Beschreibung**

Die Erfindung betrifft eine Monatsbinde mit einem Absorbierkörper, einer wasserdurchlässigen oberen Schicht, welche den Absorbierkörper sowohl an der oberen als auch den gegenüberliegenden Längsseiten bedeckt, einer wasserundurchlässigen rückseitigen Schicht, welche die untere Seite des Absorbierkörpers bedeckt und zumindest an den einander gegenüberliegenden Längsseiten der oberen Schicht mittels einer Klebschicht befestigt ist, und einer mit Öffnungen versehenen Kunststoffschaumlage, die die untere Seite der rückwärtigen Lage bedeckt und an dieser mittels einer Klebschicht befestigt ist.

Bekannte Monatsbinden, die üblicherweise verwendet werden, sind - als Befestigungsmittel zum Festlegen an der Unterwäsche - mit einer Klebschicht versehen, die auf der dem Körper der Benützerin abgewandten Seite der Binde angebracht ist, so daß jedes nachteilige Verschieben während des Gebrauchs vermieden wird.

Eine solche Binde ist aus der US-A-4 376 440 bekannt, bei der die dem Kleidungsstück zugekehrte Außenlage an ihrer Außenseite mit Klebmitteln versehen ist, die ein Anhaften der Einlage am Kleidungsstück sicherstellen sollen.

Aus der DE-A-26 44 032 ist eine Einlage bekannt, bei der der Saugkörper sowohl an der Ober- als auch an der Unterseite mit ein und derselben Umhüllungsschicht versehen ist. An der Unterseite ist zwischen dem eigentlichen Saugkörper und dieser Umhüllung eine wasserdichte Schutzfolie eingelegt, um ein Durchdringen von aufgenommener Flüssigkeit nach unten zu verhindern.

An der dem Kleidungsstück zugewandten Seite der Einlage ist eine Klebschicht aufgetragen, auf die eine perforierte Kunststoffschaumlage aufgeklebt ist.

Solche Befestigungsmittel des Standes der Technik, die auf einem Klebverfahren beruhen, werden üblicherweise an Teilbereichen der körperabgewandten Fläche der Binde vorgesehen, so daß, wenn die Benutzerin die Binde für eine längere Zeit getragen hat oder sich relativ rege bewegt hat, der übrige Bereich der Binde, der nicht an der Unterwäsche befestigt ist, sich verschoben oder verformt hat, mit der Folge, daß das Menstruum gegebenenfalls auslaufen konnte. Wenn die Befestigungsmittel über einen größeren Bereich der Oberfläche der dem Körper abgewandten Seite der Binde angeordnet sind, besteht die Gefahr, daß die Unterwäsche mit dem Klebstoff getränkt und über eine entsprechend große Fläche beschädigt wird.

Solche Befestigungen des Standes der Technik, die auf einer Kunststoffschaumlage beruhen, sind insoweit zufriedenstellend, als es das Verhindern eines Verrutschens betrifft, jedoch ist nachteilig, daß die Binde bei heruntergezogener Unterwäsche unbeabsichtigt herausfallen kann und evtl. Kleidung oder den Boden verschmutzt, da die Kunststoffschaumlage zwar einen entsprechenden Reibwiderstand in einer Ebene, die von der Unterwäsche definiert ist, aufweist, jedoch keinerlei Befestigungsfunktion in Querrichtung zu dieser Ebene besitzt, d.h. in Richtung von der Wäsche fort oder beim Abheben.

Der Erfindung liegt die Aufgabe zugrunde, eine Monatsbinde der genannten Gattung bereitzustellen, bei der die Befestigungsmittel so verbessert sind, daß die genannten Probleme behoben werden.

Die Aufgabe wird erfindungsgemäß dadurch gelöst, daß die einander gegenüberliegenden Längsränder der rückwärtigen Schicht und die Kunststoffschaumlage sich jeweils über die einander gegenüberliegenden Längsränder des Absorbierkörpers hinwegerstrecken und die Klebeschicht in den Öffnungen freiliegt.

Erfindungsgemäß weisen diese Befestigungsmittel eine aufgetragene Klebschicht in einer Dicke von 10 - 300 $\mu$m auf der dem Körper abgewandten Seite der Binde auf, sowie eine Kunststoffschaumschicht von 0,5 - 2,5 mm Dicke, die weich und elastisch ist und integral in Verbindung mit der Klebschicht steht; die Kunststoffschaumlage besitzt Öffnungen, durch welche die Oberfläche der Klebschicht teilweise frei liegt, wobei diese Öffnungen im wesentlichen oval oder kreisförmig sind, jeweils einen Durchmesser von 1 mm bis 10 mm aufweisen und in einem Abstand von 3 - 30 mm in Längs- und in Querrichtung in dieser Schaumlage angeordnet sind. Die Binde mit der daran befestigten Kunststoffschaumschicht wird gefaltet, so daß sich die Schaumstoffschicht auf der Außenseite erstreckt. Die so gefaltete Binde wird vollständig von einer Verpackungsumhüllung umgeben, ohne mit der Klebschicht, die an den Öffnungen freiliegt, an der Verpackung anzukleben, so daß die freiliegenden Flächen der Klebschicht wirksam geschützt sind.

Mit den erfindungsgemäßen Merkmalen wird eine Monatsbinde bereitgestellt, die die beim Stand der Technik aufgezeigten Nachteile beseitigt und die durch die Befestigungsmittel, die zum einen aus einer Klebschicht und zum anderen aus einer Kunststoffschaumlage bestehen, deren Vorteile aufrechterhält. Bei der erfindungsgemäßen Binde ist kein vor dem Gebrauch zu entfernender Schutzstreifen zur Abdeckung der mit Klebstoff bedeckten Flächen erforderlich, so daß eine zusätzliche Kostenersparnis erzielt wird.

Im folgenden wird die Erfindung anhand in der Zeichnung dargestellte Ausführungsbeispiele näher erläutert. Es zeigt:

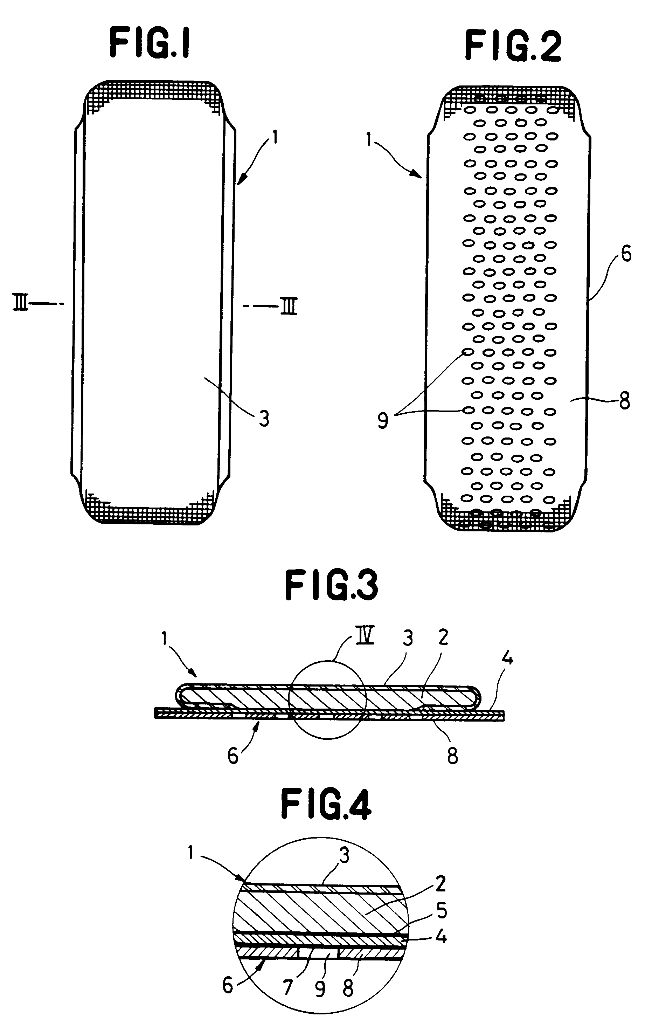

Fig. 1         eine Ansicht einer Binde von oben auf die Seite, die in Kontakt mit dem Körper kommt,

| | |
|---|---|
| Fig. 2 | eine Ansicht auf die Seite der Binde, die dem Körper abgewandt ist, |
| Fig. 3 | ein Schnitt entlang der Linie III-III in Fig. 1, |
| Fig. 4 | eine vergrößerte Darstellung eines in Fig. 3 mit einem Kreis (IV) gekennzeichneten Details, |
| Fig. 5A bis 5F | jeweils Ansichten von oben auf verschiedene Ausführungsvarianten der Befestigungsmittel, und |
| Fig. 6 | eine perspektivische Ansicht mit teilweise weggebrochenen Bereichen einer gefalteten Binde, die mit einer Einzelverpackungsumhüllung umgeben ist. |

In den Fig. 1 bis 4 ist ein Ausführungsbeispiel einer erfindungsgemäß aufgebauten Binde dargestellt. Obwohl Details der inneren Struktur nicht gezeigt sind, weist die Binde 1 einen Absorbierkörper 2, eine wasserdurchlässige obere Schicht 3 und eine wasserundurchlässige rückseitige Schicht 4 auf; dies ist auch aus dem Stand der Technik bekannt. Die obere Schicht 3 bedeckt den Absorbierkörper an ihrer Oberseite und ist mit seinen Randabschnitten um den Absorbierkörper 2 herumgelegt, so daß sie den Absorbierkörper teilweise übergreifen. Die rückwärtige Schicht 4 ist integral mit der Unterseite des Absorbierkörpers 2 und den umgefalteten Abschnitten der oberen Schicht 3 unter Zwischenschaltung einer Klebschicht 5, die auf der Oberseite der rückwärtigen Lage 4 aufgebracht worden ist, befestigt.

Die Unterseite der rückwärtigen Schicht 4 ist über ihre gesamte Fläche mit Befestigungsmitteln 6 versehen, die ihrerseits auf ihrer Unterseite eine Klebschicht 7 und eine daran angeklebte weiche elastische Kunststoffschaumlage 8 aufweisen. Die Dicke der Klebschicht 7 beträgt zwischen 10 und 300 $\mu$m, vorzugsweise 130 - 150 $\mu$m. Das Material, aus dem die Klebschicht 7 besteht, kann beispielsweise ein Copolymer eines acrylischen Esters sein, der üblicherweise zur Befestigung solcher Binden aus dem Stand der Technik an der Unterwäsche Verwendung findet. Die Kunststoffschaumlage 8 ist mit Ausnahme ihrer Randbereiche mit einer Vielzahl ovaler oder kreisförmiger Öffnungen 9 ausgebildet und weist eine Stärke von 0,5 - 2,5 mm, vorzugsweise 1 - 2 mm Dicke auf. Die Steifigkeit beträgt zwischen 5 - 20 kg/314 cm$^2$, vorzugsweise 8 - 15 kg/314 cm$^2$ (gemäß Japanischer Industriestandard JIS-k6401). Jede der Öffnungen 9 besitzt einen Durchmesser von 1 - 10 mm, vorzugsweise 2 - 5 mm, mit einem Abstand voneinander von 3 - 30 mm, vorzugsweise 5 - 15 mm. Die Kunststoffschaumlage ist üblicherweise aus einer Polyurethanlage, anderes Material, wie beispielsweise Polyethylen, Polybutadien oder EVA, kann als Lagenmaterial ebenfalls verwendet werden.

Die Klebschicht 7 würde bei einer Dicke von weniger als 10 $\mu$m keine ausreichende Klebkraft bereitstellen, bei einer Dicke von mehr als 300 $\mu$m würde der Klebstoff in den Schaum der Kunststoffschaumlage 8 eindringen und deren Funktion verschlechtern oder gar zerstören.

Die Kunststoffschaumlage 8 würde nicht ausreichend elastisch sein, um die Binde 1 gegen Deformationen abzustützen, wenn ihre Dicke weniger als 0,5 mm betrüge; die Oberfläche der Klebschicht 7, die durch die Öffnungen 9 exponiert ist, könnte nicht in einen adäquaten Kontakt mit der Unterwäsche gebracht werden, wenn die Dicke der Kunststoffschaumlage 8 2,5 mm wesentlich überschritte, dies auch dann nicht, wenn die Lage 8 unter Druck gesetzt wird. Eine Steifigkeit der Kunststoffschaumlage 8 unter 5 kg/314 cm$^2$ würde im wesentlichen den gleichen Nachteil nach sich ziehen, wie im Falle einer auf einen Wert kleiner als 0,5 mm reduzierten Dicke, eine Steifigkeit höher als 20 kg/314 cm$^2$ würde im wesentlichen ähnliche Nachteile nach sich ziehen wie im Fall einer Dicke größer als 2,5 mm.

Bei Öffnungen 9 der Lage 8 mit einem Durchmesser kleiner als 1 mm wäre im wesentlichen ein gleiches nachteiliges Ergebnis zu erwarten wie im Fall einer Dicke größer als 2,5 mm und ein Durchmesser größer als 10 mm würde eine Situation herbeiführen, bei der die Steifigkeit der Kunststoffschaumlage 8 ungenügend wäre, um eine Deformation der Binde 1 zu verhindern, und wenn die Binde mit einer einzelnen Verpackungshülle, wie nachfolgend näher erläutert, bedeckt ist, würde die Oberfläche der Klebschicht 7, die durch die Öffnungen 9 hervortritt, an der Umhüllung festkleben und der Klebstoff würde von den Abschnitten der Klebschicht 7, die auf diese Weise an der Hülle festklebt, abgeschält werden, mit dem Ergebnis, daß die Binde mit einem entfernbaren Schutzstreifen für die durch die Öffnungen 9 exponierte Klebschicht 7 bedeckt werden müßte, wie es der Fall ist bei Befestigungsmitteln nach dem Stand der Technik, die auf einer Klebung basieren, und zwar getrennt von der Verpackungshülle. Wenn die Öffnungen dieser Schaumlage in einem Abstand von weniger als 3 mm angeordnet sind, ist die Steifigkeit der Schaumstofflage 8 nicht ausreichend, um die Binde gegen mögliche Verformungen zu schützen, ist der Abstand jedoch größer als als 30 mm, so ist die Klebschicht 7 nicht wirksam.

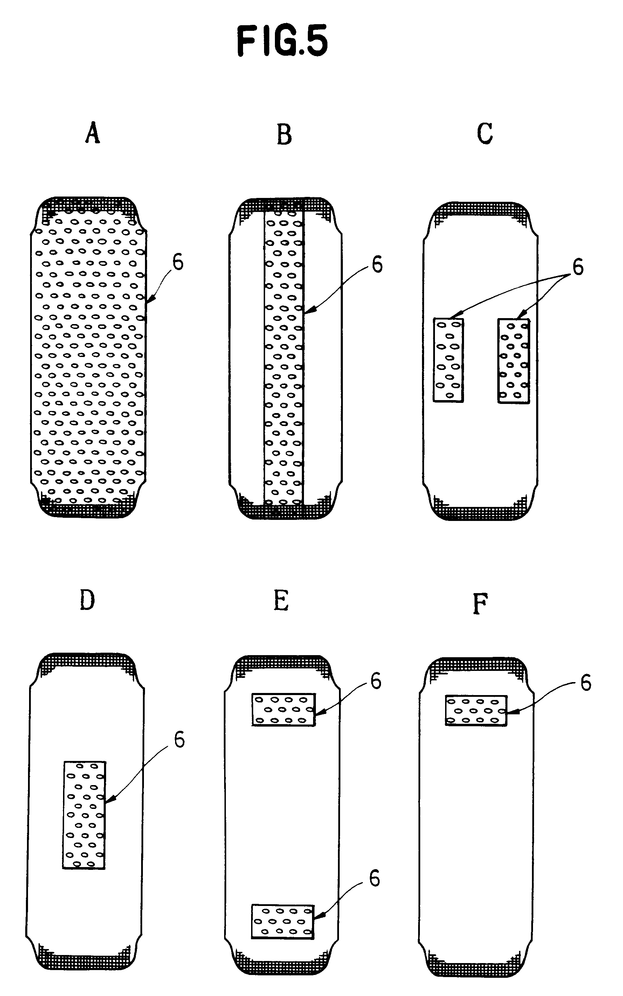

In den Fig. 5A bis 5F sind Beispiele von Anordnungen der Befestigungsmittel 6 angegeben. In Fig. 5A sind Öffnungen 9 über die gesamte Unterseite der Binde verteilt vorgesehen. Bei der in Fig. 5B dargestellten Binde erstrecken sich die Öffnungen 9 lediglich über einen schmalen Längsstreifen etwa in der Längssymmetrieachse. In Fig. 5C sind die Öffnungen lediglich in der Mitte der Binde, jeweils im

**EP 0 229 639 B1**

Abstand von der Längssymmetrieachse vorgesehen. Die in Fig. 5D dargestellte Variante entspricht in etwa derjenigen, die in Fig. 5B gezeigt ist, jedoch sind bei dieser Befestigungsanordnung die Endbereiche frei von Öffnungen. Im Gegensatz dazu sind in dem in Fig. 5E dargestellten Ausführungsbeispiel die Öffnungen lediglich im Bereich der Bindenenden vorgesehen, schließlich ist in Fig. 5F der Bereich, an dem Befestigungsöffnungen vorgesehen sind, lediglich auf ein Ende der Binde beschränkt.

Es versteht sich, daß die Befestigungsmittel 6 über die gesamte Fläche oder an gewünschten Teilbereichen der Binde so angeordnet sind, daß sie außerhalb jeglichen Kontakts mit der Haut des Benutzers sind.

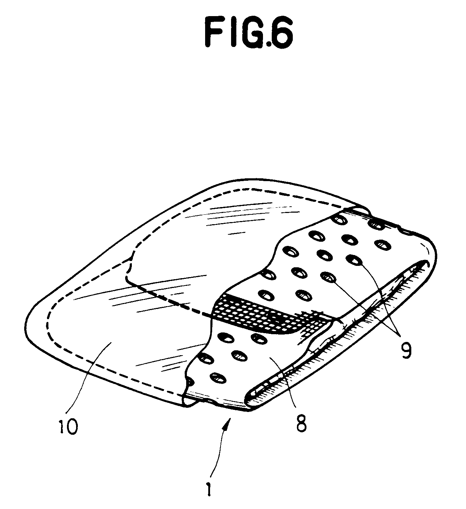

Wie aus Fig. 6 zu erkennen ist, wird zum Verpacken die Binde, die mit den Befestigungsmitteln versehen ist, gefaltet, so daß die Kunststoffschaumlage 8 sich auf der Außenseite befindet und in diesem Zustand von der Einzelverpackungshülle 10 bedeckt ist. Die Hülle 10 besteht vorzugsweise aus einem flexiblen Kunststoffilm, da ein solcher Film leicht entlang seiner Ränder heißversiegelbar ist, um eine solche Hülle zu bilden; sollte die Innenfläche der Umhüllung an der Oberfläche der Klebschicht 7, die durch die Öffnungen 9 exponiert ist, ankleben, so wird das Material der Hülle teilweise abgeschält, so daß der gewünschte Klebeffekt der Klebschicht 7 erhalten bleibt. Vorzugsweise besteht die Verpackungshülle an ihrer Innenseite aus einem faserigen Vlies. In einem solchen Fall kann die Verpackungshülle 10 an der Klebschicht 7 Fasern zurücklassen. Obwohl die Umhüllung 10 als teilweise aufgebrochen gezeichnet ist, versteht es sich, daß die Umhüllung die Binde vollständig bedeckt.

4

| Beispiel | Dicke der Klebschicht (µm) | Kunststoffschaumlage | | | | Klebeffekt | Verhinderungseffekt gegen Verformung |
|---|---|---|---|---|---|---|---|
| | | Dicke (mm) | Öffnung (mm) | Öffnungsabstand (mm) | Steifigkeit (Kg/314cm²) | | |
| No. 1 | 30 | 1 | 2,5 | 6 | 10 | O | O |
| No. 2 | 5 | 1 | 2,5 | 6 | 10 | X | O |
| No. 3 | 300 | 1 | 2,5 | 6 | 10 | O | X |
| No. 4 | 30 | 0,5 | 2,5 | 6 | 10 | O | X |
| No. 5 | 30 | 2,5 | 2,5 | 6 | 10 | X | O |
| No. 6 | 30 | 1 | 0,8 | 6 | 10 | X | O |
| No. 7 | 30 | 1 | 11,0 | 6 | 10 | O | X |
| No. 8 | 30 | 1 | 2,5 | 3 | 10 | O | X |
| No. 9 | 30 | 1 | 2,5 | 30 | 5 | X | O |
| No. 10 | 30 | 1 | 2,5 | 6 | 5 | O | X |
| No. 11 | 30 | 1 | 2,5 | 6 | 20 | X | O |

BEMERKUNGEN : ◯ = gut ✕ = schlecht

Die erfindungsgemäß aufgebaute Binde wird für den Gebrauch mit der Unterseite mit der Kunststoffschaumlage 8 nach unten gerichtet gegen die Unterwäsche gedrückt und auf diese Weise befestigt. Wenn die Binde an der Kleidung festgelegt ist, bleibt die eigentliche Binde unter Zwischenlage der Klebschicht, die durch die Öffnungen 9 exponiert ist, an der Wäsche kleben und wird daran gehindert, sich aus einer Ebene zu bewegen, die von der Wäsche definiert wird. Zum einen wird sie dabei durch den Klebeffekt in Lage gehalten, zum anderen unterstützt der Reibwiderstand der Kunststoffschaumlage 8 in bezug auf die Wäsche dieses Inlagehalten und widersteht einer Verschiebung in dieser Ebene. Auf diese Weise werden die Probleme, die bei Binden nach dem Stand der Technik auftreten, vollständig bewältigt.

Bei der erfindungsgemäß aufgebauten Binde sind verschiedene Faktoren, wie beispielsweise die Dicke der Kunststoffschaumlage 8 und die Größe der Öffnungen 9 optimiert. Die Binde wird so gefaltet, daß die Kunststoffschaumlage 8 sich auf der Außenseite erstreckt, so daß eine einzelne Verpackungsumhüllung 10 die Binde vollständig umschließt und es nicht notwendig ist, die Binde mit einem separat vorgesehenen abziehbaren Streifen abzudecken, um die Oberfläche der Klebschicht 7, die durch die Öffnungen 9 freiliegt, zu schützen.

## Patentansprüche

**1.** Monatsbinde mit einem Absorbierkörper (2), einer wasserdurchlässigen oberen Schicht (3), welche den Absorbierkörper (2) sowohl an der oberen als auch den gegenüberliegenden Längsseiten bedeckt, einer Wasserundurchlässigen rückseitigen Schicht (4), welche die untere Seite des Absorbierkörpers (2) bedeckt und zumindest an den einander gegenüberliegenden Längsseiten der oberen Schicht (3) mittels einer Klebschicht (5) befestigt ist, und einer mit Öffnungen (9) versehenen Kunststoffschaumlage (8), die die untere Seite der rückwärtigen Lage (4) bedeckt und an dieser mittels einer Klebschicht (7) befestigt ist, dadurch **gekennzeichnet**, daß die einander gegenüberliegenden Längsränder der rückwärtigen Schicht (4) und die Kunststoffschaumlage (8) sich jeweils über die einander gegenüberliegenden Langsränder des Absorbierkörpers (2) hinweg erstrecken und die Klebeschicht (7) in den Öffnungen freiliegt.

## Claims

**1.** A sanitary towel with an absorbent body (2), a water-permeable upper layer (3) which covers the absorbent body (2) both on the upper side and on both of the opposed longitudinal sides, a waterproof rear-side layer (4) which covers the underside of the absorbent body (2) and is fixed by means of an adhesive layer (5) at least to the mutually opposed longitudinal edges of the upper layer (3), and a plastics foam layer (8) provided with openings (9), which covers the lower side of the rear-side layer and is fixed thereto by means of an adhesive layer (7), characterized in that the mutually opposed longitudinal edges of the rear-side layer (4) and the plastics foam layer (8) extend beyond the mutually opposed longitudinal edges of the absorbent body (2) and the adhesive layer (7) is exposed in the openings.

## Revendications

**1.** Serviette hygiénique comportant un corps absorbant (2), une couche supérieure (3) perméable à l'eau, qui recouvre le corps absorbant (2) tant sur le dessus que sur les côtés longitudinaux opposés, une couche arrière (4), imperméable à l'eau, qui recouvre le dessous du corps absorbant (2) et qui est fixée par une couche adhésive (5) au moins sur les côtés longitudinaux mutuellement opposés de la couche supérieure (3), et une couche de mousse synthétique (8) pourvue d'ouvertures (9), qui recouvre le dessous de la couche arrière (4) et qui est fixée à cette dernière par une couche adhésive (7), caractérisée en ce que les bords longitudinaux mutuellement opposés de la couche arrière (4) et la couche de mousse synthétique (8) se prolongent respectivement au-delà des bords longitudinaux mutuellement opposés du corps absorbant (2) et en ce que la couche adhésive (7) est absente dans les ouvertures.

# FIG.1

# FIG.2

# FIG.3

# FIG.4

# FIG.5

A

B

C

6

6

6

D

E

F

6

6

6

6

# FIG.6